# EUROPÄISCHE PATENTSCHRIFT

(11) **EP 1 171 107 B1**
(45) Veröffentlichungstag und Bekanntmachung des Hinweises auf die Patenterteilung: **08.12.2004**
(21) Anmeldenummer: 00907468.3
(22) Anmeldetag: 13.01.2000
(51) Int. Cl.: A61K 31/14, A61P 27/02

(54) **VERWENDUNG EINER PHARMAZEUTISCHEN ZUSAMMENSETZUNG ZUR HERSTELLUNG EINES MEDIKAMENTES ZUR BEHANDLUNG VON DURCH BAKTERIEN, VIREN, PILZE, HEFEN UND/ODER PROTOZOEN VERURSACHTEN AUGENERKRANKUNGEN**
USE OF A PHARMACEUTICAL COMPOSITION IN THE MANUFACTURE OF A MEDICAMENT FOR THE TREATMENT OF PATHOLOGICAL CONDITIONS OF THE EYE CAUSED BY BACTERIA, VIRUSES, FUNGI, YEASTS AND PROTOZOA
UTILISATION D'UN COMPOSITION PHARMACEUTIQUE POUR LA FABRICATION D'UN MEDICAMENT SERVANT A TRAITER DES ETATS OPHTHALMIQUES PATHOLOGIQUES INDUITS PAR BACTERIES, VIRUS, CHAMPIGNONS, LEVURES ET PROTOZOAIRES

(30) Priorität: 22.04.1999 DE 19918324
(43) Veröffentlichungstag der Anmeldung: 16.01.2002
(73) Patentinhaber: Dr. GERHARD MANN chem.-pharm. Fabrik GmbH, D-13581 Berlin (DE)
(72) Erfinder: CLAUS-HERZ, Gudrun, D-14167 Berlin (DE); KESSLER, Christoph, D-10625 Berlin (DE); BELLMANN, Günther, D-13593 Berlin (DE)
(74) Vertreter: Maiwald Patentanwalts GmbH
(86) Internationale Anmeldenummer: PCT/EP2000/000191
(87) Internationale Veröffentlichungsnummer: WO 2000/064429

(56) Entgegenhaltungen:
- EP-A- 0 306 984
- EP-A- 0 308 564
- WO-A-94/15597
- WO-A-95/19766
- WO-A-99/25190
- DE-A- 2 739 661
- FR-A- 2 616 065
- DATABASE WPI Section Ch, Week 199830 Derwent Publications Ltd., London, GB; Class A96, AN 1998-343209 XP002136346 & JP 10 130156 A (TEIKA SEIYAKU KK), 19. Mai 1998 (1998-05-19)
- DATABASE WPI Section Ch, Week 198945 Derwent Publications Ltd., London, GB; Class B05, AN 1989-329469 XP002136347 & JP 01 246227 A (SANTEN PHARM CO LTD), 2. Oktober 1989 (1989-10-02)
- DATABASE WPI Section Ch, Week 199612 Derwent Publications Ltd., London, GB; Class A96, AN 1996-112632 XP002136348 & JP 08 012572 A (KOZAKAI YG), 16. Januar 1996 (1996-01-16)
- DATABASE WPI Section Ch, Week 199724 Derwent Publications Ltd., London, GB; Class B05, AN 1997-267718 XP002136349 & JP 09 095457 A (SUNSTAR CHEM IND CO LTD) , 8. April 1997 (1997-04-08)
- "Martindale" 1996 , ROYAL PHARMACEUTICAL SOCIETY , LONDON XP002136345 "Benzalkonium chloride" "Benzododecinium bromide" Seite 1117 -Seite 1119
- STARR M.B. ET AL: "Antimicrobial prophylaxis for ophthalmic surgery." SURVEY OF OPHTHALMOLOGY, (1995) 39/6 (485-501). , XP000886466

## Beschreibung

Die Erfindung betrifft die Verwendung einer pharmazeutischen Zusammensetzung wirksam gegen durch Bakterien, Viren, Pilze, Hefen und/oder Protozoen verursachte Krankheitszustände, insbesondere in Form einer wäßrigen Lösung, eines tropfbaren Gels, einer Salbe oder dergleichen. Außerdem betrifft die Erfindung die Verwendung der pharmazeutischen Zusammensetzung zur Herstellung eines Arzneimittels zur Behandlung der durch die vorgenannten Krankheitserreger verursachten Krankheitszustände. Ferner betrifft die Erfindung die Verwendung der pharmazeutisch wirksamen Stoffe zur Herstellung von ophthalmischen Zusammensetzungen.

Im Stand der Technik sind zahlreiche antibiotisch wirksame Substanzen aus Pflanzen und Tieren sowie chemische oder biosynthetisch hergestellt Derivate davon bekannt, die eine Wachstumshemmung von Mikroorganismen bewirken. Beispiele hierfür sind Ofloxacin und Ciprofloxacin. So werden beispielsweise Ofloxacin und Ciprofloxacin als Antibiotika in der Ophthalmologie verwendet. Bekannte Präparate, die diese Wirkstoffe enthalten, sind Floxal® der Dr. Mann Pharma und Ciloxan® der Firma Alcon.

Ferner sind eine Vielzahl von Virustatika bekannt. Zu den gebräuchlichsten Virustatika gehören Aciclovir, Ganciclovir, Vidarabin und Interferon. Bekanntermaßen treten bei häufiger Anwendung von Antibiotika und Virustatika bei den zu behandelnden Patienten Resistenzen gegen diese Wirkstoffe auf. Dies hat den großen Nachteil, daß Bakterien und/oder Virus bedingte Krankheitszustände bei Verabreichung von Wirkstoffen, gegen die der Patient bereits eine Resistenz ausgebildet hat, nicht mehr vollständig ausgeheilt werden können, so daß sogar schlimmstenfalls eine Verschlechterung des Krankheitszustandes eintritt.

Es ist außerdem Stand der Technik, daß Benzalkoniumchlorid in pharmazeutischen Zusammensetzungen zur Verwendung als Konservierungsmittel geeignet ist, insbesondere um die Sterilität und die Lagerfähigkeit von ophthalmischen Präparaten, die nicht zum sofortigen Gebrauch bestimmt sind, zu erhöhen.

Aufgabe der vorliegenden Erfindung ist es, eine pharmazeutische Zusammensetzung mit verbesserten bakteriziden und viruziden Eigenschaften zur Verfügung zu stellen, die auch gegen Pilze, Hefen und/oder Protozoen wirksam ist.

Es wurde nun überraschend gefunden, daß Alkylbenzyldimethylammonium-Salz ein pharmazeutisch hoch wirksames Bakterizid und Viruzid ist, das darüber hinaus auch gegen Pilze, Hefen und Protozoen wirkt.

Obwohl die Verwendung von Benzalkoniumsalzen in pharmazeutischen Zusammensetzungen als Konservierungsmittel seit langem bekannt ist, wurde bisher nicht erkannt, daß Benzalkoniumsalz bzw. Benzalkoniumsalze als pharmazeutischer Wirkstoff gegenüber einer Reihe von Antibiotika eine gesteigerte Aktivität aufweist und gegen durch die vorgenannten Erreger verursachten Krankheitszustände hoch wirksam ist und praktisch keine Resistenzen ausbildet.

Die erfindungsgemäß gestellte Aufgabe wird insbesondere durch die Verwendung einer pharmazeutischen Zusammensetzung zur Herstellung eines Medikaments zur Behandlung von durch Bakterien, Viren, Pilze, Hefen und/oder Protozoen verursachte Augenerkrankungen gelöst, indem diese pharmazeutische Zusammensetzung als alleinigen pharmazeutisch wirksamen Stoff eine Verbindung der allgemeinen Formel oder ein Gemisch davon: 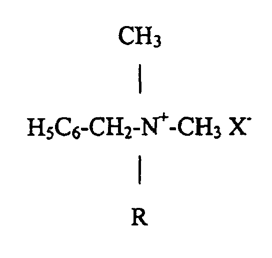 umfaßt, in der R ein Alkylrest von C₈-C₁₈, vorzugsweise C₁₂ und X ein Anion, vorzugsweise ein Halogenid ist, wobei die pharmazeutische Zusammensetzung ggf. einen Puffer enthält, vorzugsweise einen Phosphat- oder Boratpuffer sowie ggf. weitere Zusätze wie Wasser, Konservierungsmittel, EDTA und/oder Isotonierungsmittel umfaßt.

Weitere vorteilhafte Verwendungen der pharmazeutischen Zusammensetzungen sind in den Unteransprüchen aufgeführt.

Besonders bevorzugt ist wenn X ein Chlorid und/oder Bromid ist.

Als Benzalkoniumchloride, die unter die oben angegebene allgemeine Formel fallen, werden Gemische von Alkylbenzyldimethylammoniumchloriden mit unterschiedlichen Alkyl-Resten im C-Zahlenbereich von 8-18 bezeichnet. Die Verwendung solcher Gemische hat den Nachteil, daß insbesondere bei wiederholter Anwendung oder bei Verabreichung über einen längeren Zeitraum, insbesondere bei einer verlängerten Verweilzeit auf der Haut- und Schleimhaut Irritationen wie Rötungen etc. auftreten können. So können Benzalkoniumchlorid-Gemische bei Verwendung als Konservierungsmittel am Auge Irritationen und sogar Gewebeschädigungen verursachen. Es ist daher erfindungsgemäß bevorzugt, eine pharmazeutische Zusammensetzung zu verwenden, die als pharmazeutisch wirksamen Stoff Benzyllauryldimethylammonium-Salz aufweist. Besonders vorteilhaft ist die Verwendung von Benzyllauryldimethylammoniumchlorid und/oder Benzyllauryldimethylammoniumbromid, wobei das Chloridsalz am meisten bevorzugt ist.

Besonders vorteilhaft verwendbar sind Arzneimittelzusammensetzungen, die Benzalkoniumsalz oder Benzalkoniumsalze als alleinigen pharmazeutisch wirksamen Stoff enthalten.

Eine weitere erfindungsgemäß bevorzugt verwendbare Ausfuhrungsform umfaßt eine pharmazeutische Zusammensetzung, die als pharmazeutisch wirksamen Stoff Alkylbenzyldimethylammonium-Salz in einer Konzentration von ≤ 1 Gew.-%, bezogen auf die pharmazeutische Gesamtzusammensetzung, vorzugsweise im Bereich zwischen 0,00005 Gew.-% - 0,2 Gew.-% und besonders bevorzugt im Bereich von zwischen 0,001 Gew.-% - 0,05 Gew.-% umfaßt.

In einer weiteren erfindungsgemäß bevorzugt verwendbaren Ausführungsform umfaßt die pharmazeutische Zusammensetzung Substanzen mit osmotischen Eigenschaften wie Natrium Chlorid, Natriumnitrat, Kaliumnitrat, Glukose, Glycerol und/oder Sorbitol.

Besonders bevorzugt ist die erfindungsgemäß verwendbare pharmazeutische Benzalkoniumsalz-Zusammensetzung unviskosiert.

Weitere verwendbare pharmazeutische Zusammensetzungen können auch eine Viskosierung aufweisen. Bevorzugt ist, daß die viskosierten pharmazeutischen Zusammensetzungen wenigstens einen die Viskosität erhöhenden Stoff wie synthetisches oder natürliches Polymer, vorzugsweise ein Cellulosederivat, Hydroxypropylmethylcellulose, Carbomer, Xanthan, Dextran, Carboxyvinylpolymer, insbesondere Carboxypolymethylen und/oder ein Ethylen-Maleinanhydrid-Copolymer und/oder Hydroxyethylcellulose oder Derivate sowie Mischungen davon, besonders bevorzugt in wäßriger Lösung oder Dispersion umfaßt.

Weitere erfindungsgemäß verwendbare pharmazeutische Zusammensetzungen liegen in Form einer unviskosierten Lösung vor. Die Lösungen können auch eine Viskosierung aufweisen, wobei diese Lösungen vorzugsweise eine Viskosität im Bereich von zwischen 0-7000 mPa·s, vorzugsweise zwischen 2-8 mPa·s aufweisen. Erfindungsgemäß verwendbar sind außerdem Gele, vorzugsweise mit einer Viskosität im Bereich von zwischen 1000-7000 mPa·s.

Außerdem sind ophthalmische Zusammensetzungen erfindungsgemäß verwendbar, die in Form eines tropfbaren Gels, einer Salbe oder einer Lösung vorliegen und als pharmazeutisch wirksamen Stoff Alkylbenzyldimethylammonium-Salz aufweisen. Gegebenenfalls können diese ophthalmischen Zusammensetzungen weitere Wirkstoffe und übliche Zusätze wie Isotonierungsmittel und Substanzen zur Einstellung des pH-Wertes, wie Phosphatpuffer, Boratpuffer, Alkylhydroxid und/oder Säure aufweisen.

Die vorgenannten pharmazeutischen Zusammensetzungen sind insbesondere zur Behandlung von durch Bakterien, Viren, Pilze, Hefen und/oder Protozoen bedingter Krankheitszustände geeignet. Insbesondere lassen sich die als pharmazeutisch wirksamen Stoff Alkylbenzyldimethylammonium-Salz enthaltenen Zusammensetzungen zur Behandlung von durch Bakterien, Viren, Pilze, Hefen und/oder Protozoen verursachte Krankheitszustände am und/oder im Auge verwenden. Als pharmazeutischer Wirkstoff in ophthalmischen Zusammensetzungen sind insbesondere Benzyllauryldimethylammoniumbromid und das Benzyllauryldimethylammoniumchlorid bevorzugt verwendbar.

### Untersuchung verschiedener Testsubstanzen

### Eingesetzte Teststämme:

Escherichia (E.coli)
Staphylococcus (S.aureus)
Streptococcus (Sc.)
Pneumoniae
Pseudomonas (P.aeruginosa)
Staphylococcus (S. epidermidis)
Haemophilus (H. influenzae)

Bezugsquelle: Deutsche Sammlung für Mikroorganismen und Zellkulturen (DSMZ)

### Anzucht der Teststämme:

Die verschiedenen Testkeime wurde jeweils aus einem Lyophilisat in NaCl-Peptonpuffer homogenisiert und auf Caseinpepton-Sojymehlpepton. (Caso-) Agar (Fa. bioMérieux; Art. Nr. 43019) bez. Kochblut-Agar (Fa. bioMérieux; Art. Nr. 43109) subkultiviert. Die Teststämme wurden vor Versuchsbeginn durch Erstellen eines biochemischen Profils mittels ATB/Vitek-System (Fa. bioMérieux, Nürtingen) auf Reinheit und Identität überprüft.

### Ausgangskeimzahl:

Die Ausgangskeimzahl wurde im Oberflächenverfahren mit Hilfe eines Spiralplaters (Fa. Meintrup, Lähden) ermittelt und ist im einzelnen den Tabellen 1-12 zu entnehmen.

| **Testsubstanzen:** | |
|---|---|
| a. | Benzalkoniumchlorid 0,0025% |
| b. | Benzalkoniumchlorid 0,001 % |
| c. | Benzalkoniumchlorid 0,006% |
| d. | Benzododeciniumchlorid 0,005% |
| e. | Benzododeciniumchlorid 0,01 % |
| f. | Benzododeciniumchlorid 0,02% |
| g. | Floxal® |
| h. | Ciloxan® |
| i. | Ofloxacin 0,3% |
| j. | Ciprofloxacin HCl 0,35% |

### Versuchsdurchführung:

Die verschiedenen Testsubstanzen a. bis j. wurden jeweils zu 5 ml in sterile Zentrifugenröhrchen abgefüllt und mit ca. 10⁵ KBE^{*}/ml (* = koloniebildende Einheiten) der oben genannten Testkeime kontaminiert. Pro Substanz wurde für jeden Testkeim und jede zu prüfende Testzeit ein separater Ansatz angeimpft, d.h.10 Testsubstanzen zu jeweils 7 Testzeiten und 6 Testkeime (= insgesamt 502 Testansätze). Nach 2, 6, 8, 24, 32, 48 und 72 Stunden wurde dann der jeweilige Testansatz 10 Minuten bei 3000 rpm abzentrifugiert. Der Überstand wurde dekantiert und das Pellet in 5 ml NaCl Peptonpuffer aufgenommen und resuspendiert. Die Suspension wurde erneut 10 Minuten bei 3000 rpm abzentrifugiert und 5 ml NaCl-Peptonpuffer aufgenommen und mittels Vortex homogenisiert.

### Quantitative Keimzahlbestimmung:

Die anschließende Keimzahlbestimmung erfolgte im Oberflächenverfahren durch Ausspiralisieren eines 10011 Aliquots auf Caso-Agar bzw. für H. influenzae auf Kochblut-Agar. Die Platten wurden bei 33±2°C für max. 4 Tage bebrütet. Die Nachweisgrenze des Verfahrens liegt bei 100 KBE/ml.

### Qualitative Untersuchung:

Jeweils 2 ml der Testansätze wurden in 20 ml Caso-Bouillon oder für H. influenzae, in Fildes-Medium der Firma Becton Dickinson, ARt. Nr. 0349-739, angereichert und ebenfalls maximal 4 Tage bei 33± 2°C bebrütet. Ein negatives Ergebnis der Anreicherung belegt die Elimination der inokulierten Keime.

Die Ergebnisse der Keimzählung sowie der Anreicherungen sind nachfolgend in den Tabellen 1 bis 10 zusammengestellt.

### Versuchsergebnisse:

| **Testsubstanz** | | | |
|---|---|---|---|
| a) | **Benzalkoniumchlorid 0,0025 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 × 10⁵ KBE/ml |
| C | Sc. pneumoniae | 3,7 × 10⁵ KBE/ml | 2,4 × 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 1**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - - | | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. | | | | | | |

| **Testsubstanz** | | | |
|---|---|---|---|
| b) | **Benzalkoniumchlorid 0,001 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 2**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. | | | | | | |

| **Testsubstanz** | | | |
|---|---|---|---|
| c) | **Benzalkoniumchlorid 0,006 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBF,/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 × 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 3**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind praktisch 2 h nach Inokulation der Zubereitung auch qualitativ nicht mehr nachweisbar. | | | | | | |

| **Testsubstanz** | | | |
|---|---|---|---|
| d) | **Benzalkoniumchlorid 0,005 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 × 10⁵ KBE/ml | 2,3 x 10⁵ KBE/ml |
| C | Sc. pneumoniae | 3,7 × 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 4**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: | | | | | | |

| **Testsubstanz** | | | |
|---|---|---|---|
| e) | **Benzododeciniumchlorid 0,01 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 5**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. | | | | | | |

| **Testsubstanz** | | | |
|---|---|---|---|
| f) | **Benzododeciniumchlorid 0,02 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 6**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. | | | | | | |

| **Vergleichstest** | | | |
|---|---|---|---|
| g) | **Floxal®** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 × 10⁵ KBE/ml |
| C | Sc. pneumoniae | 3,7 x 10⁵ KBE/ml | 2,4 x 14⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 7**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. Floxal® = Zusammensetzung: 1 ml enth. Ofloxacin 3 mg. Weitere Bestandteile: 0,0025 Gew.-% Benalkoniumchlorid als Konservierungsmittel, Natriumchlorid, Salzsäure und Natriumhydroxid zur pH-Wert Einstellung und Wasser. | | | | | | |

| **Vergleichstest** | | | |
|---|---|---|---|
| h) | **Ciloxan®** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 × 10⁵ KBE/ml |
| F | H.influenzae | 2,8 x 10⁵ KBE/ml | 1,4 x 10⁵ KBE/ml |

**Tabelle 8**

| **Zeit** | **A** | **B** | **C** | **D** | **E** | **F** |
|---|---|---|---|---|---|---|
| 2 h | <100 | <100 | <100 | <100 | <100 | <100 |
| | - | - | - | - | - | - |
| 4 h | <100 | <100 | <100 | <100 | <100 | <100 |
| | - | - | - | - | - | - |
| 6 h | <100 | <100 | <100 | <100 | <100 | <100 |
| | - | - | - | - | - | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind bereits 2 h nach Inokulation der Zubereitung auch qualitativ nicht mehr nachweisbar. Ciloxan® = Zusammensetzung: 1 ml enth. 3,5 mg Ciprofloxacin-HCl (3,5 mg = 3 mg Ciprofloxacin). Weitere Bestandteile sind 0,006 Gew.-% Benzalkoniumchlorid als Konservierungsmittel, 0,06 mg Edetinsäure, Dinatriumsalz 2H₂O, Mannitol, Essigsäure, Natriumacetat und Wasser. | | | | | | |

| **Vergleichstest** | | | |
|---|---|---|---|
| i) | **Ofloxacin 0,3 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 x 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 9**

| **Zeit** | **A** | **B** | **C** | **D** | **E** | **F** |
|---|---|---|---|---|---|---|
| 2 h | <100 | <100 | <100 | <100 | <100 | <100 |
| | - | + | - | - | + | - |
| 4 h | <100 | <100 | <100 | <100 | <100 | <100 |
| | - | + | + | + | + | - |
| 6 h | <100 | <100 | <100 | <100 | <100 | <100 |
| | - | + | - | - | + | - |
| 8 h | <100 | <100 | <100 | <100 | <100 | <100 |
| | - | - | - | - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind spätestens 24 h nach Inokulation der Zubereitung auch qualitativ nicht mehr nachweisbar. | | | | | | |

| **Vergleichstest** | | | |
|---|---|---|---|
| j) | **Ciprofloxacin HCl 0,35 Gew.-%** | | |
| | **Testkeime** | **Ausgangskeimzahl** | **Positivkontrolle (angeimpfte Pufferlösung)** |
| A | E.coli | 2,7 x 10⁵ KBE/ml | 2,5 x 10⁵ KBE/ml |
| B | S.aureus | 2,6 x 10⁵ KBE/ml | 2,3 x 10⁵ KBE/ml |
| C | Sc. pneumoniae | 3,7 x 10⁵ KBE/ml | 2,4 × 10⁵ KBE/ml |
| D | P.aeruginosa | 2,7 x 10⁵ KBE/ml | 1,4 x 10⁵ KBE/ml |
| E | S.epidermidis | 2,1 x 10⁵ KBE/ml | 1,9 x 10⁵ KBE/ml |
| F | H.influenzae | 2,8 x 10⁵ KBE/ml | 1,4 x 10⁵ KBE/ml |

**Tabelle 10**

| **Zeit** | **A** | **B** | **C** | **D** | **E** | **F** |
|---|---|---|---|---|---|---|
| 2 h | <100 | <100 | <100 | <100 | <100 | <100 |
| | - | + | - | - | + | - |
| 4 h | <100 | <100 | <100 | <100 | <100 | <100 |
| | - | + | - | - | + | - |
| 6 h | <100 | <100 | <100 | <100 | <100 | <100 |
| | - | + | - | - | + | - |
| 8 h | <100 | <100 | <100 | <100 | <100 | <100 |
| | - | + | - | - - | - | - |
| 24 h | <100 | <100 | <100 | <100 | <100 | <100 |
| | - | - | - | - | - | - |
| 32 h | <100 | <100 | <100 | <100 | <100 | <100 |
| | - | - | - | - | - | - |
| 48 h | <100 | <100 | <100 | <100 | <100 | <100 |
| | - | - | - | - | - | - |
| 72 h | <100 | <100 | <100 | <100 | <100 | <100 |
| | - | - | - | - | - | - |
| Ergebnis der Reisolierung (in KBE/ml sowie qualitiativ (+/-) nach Anreicherung: **Beurteilung:** Alle Testkeime sind spätestens 24 h nach Inokulation der Zubereitung auch qualitativ nicht mehr nachweisbar. | | | | | | |

Die Versuchsergebnisse der Tabellen 1 bis 6 belegen eindeutig, daß Benzalkoniumchlorid a) bis c) und Benzododeciniumchlorid d) bis f) gegenüber den pharmazeutischen Wirkstoffen Ofloxacin und Ciprofloxacin HCI eine deutlich verbesserte antibiotische Wirkung aufweisen. So sind bereits alle Testkeime bei den Testsubstanzen a) bis f) 2 Stunden nach Inokulation der Zubereitung auch qualitativ nicht mehr nachweisbar. Dagegen sind bei der Testsubstanz Ofloxacin erst 24 Stunden nach Inokulation der Zubereitung die Testkeime auch qualitativ nicht mehr nachweisbar. Bei Verwendung der Testsubstanz Ciprofloxacin HCl konnten ebenfalls erst nach 24 Stunden nach Inokulation der Zubereitung keine Testkeime qualitativ mehr nachgewiesen werden. Darüber hinaus belegen die Vergleichsversuche mit den Testsubstanzen g) und h), daß Benzalkoniumchlorid a) bis f) eine höhere pharmazeutische Wirksamkeit besitzt als Ofloxacin und Ciprofloxacin.

## Patentansprüche

1. Verwendung einer pharmazeutischen Zusammensetzung zur Herstellung eines Medikaments zur Behandlung von durch Bakterien, Viren, Pilze, Hefen und/oder Protozoen verursachte Augenerkrankungen
**dadurch gekennzeichnet, daß** die pharmazeutische Zusammensetzung als alleinigen pharmazeutisch wirksamen Stoff eine Verbindung der allgemeinen Formel oder ein Gemisch davon: 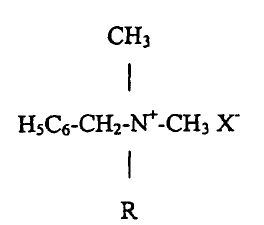 umfaßt, in der R ein Alkylrest von C₈-C₁₈, vorzugsweise C₁₂ und X ein Anion, vorzugsweise ein Halogenid ist, wobei die pharmazeutische Zusammensetzung ggf. einen Puffer, vorzugsweise einen Phosphat- oder Boratpuffer sowie ggf. weitere Zusatzstoffe wie Wasser, Konservierungsmittel, EDTA und/oder Isotonierungsmittel aufweist.

2. Verwendung der pharmazeutischen Zusammensetzung nach Anspruch 1,
**dadurch gekennzeichnet, daß** das Anion, vorzugsweise ein Chlorid und/oder Bromid ist.

3. Verwendung der pharmazeutischen Zusammensetzung nach Anspruch 1 oder 2,
**dadurch gekennzeichnet, daß** das Alkylbenzyldimethylammonium-Salz in einer Konzentration von ≤ 1-Gew.-%, bezogen auf die pharmazeutische Gesamtzusammensetzung, vorzugsweise im Bereich zwischen 0,00005 Gew.-% - 0,2 Gew.-% und besonders bevorzugt im Bereich von zwischen 0,001 Gew.-% - 0,05 Gew.-%, vorliegt.

4. Verwendung der pharmazeutischen Zusammensetzung nach einem der Ansprüche 1 bis 3,
**dadurch gekennzeichnet, daß** die pharmazeutische Zusammensetzung Substanzen mit osmotischen Eigenschaften wie Natriumchlorid, Natriumnitrat, Kaliumnitrat, Glukose, Glycerol und/oder Sorbitol umfaßt.

5. Verwendung der pharmazeutischen Zusammensetzung nach einem der Ansprüche 1 bis 4,
**dadurch gekennzeichnet, daß** die pharmazeutische Zusammensetzung unviskosiert ist, vorzugsweise wenigstens einen die Viskosität erhöhenden Stoff wie synthetisches oder natürliches Polymer, vorzugsweise ein Cellulosederivat, Hydroxypropylmethylcellulose, Carbomer, Xanthan, Dextran, Carboxyvinylpolymer, insbesondere Carboxypolymethylen und/oder ein Ethylen-Maleinanhydrid-Copolymer und/oder Hydroxyethylcellulose oder Derivate sowie Mischungen davon, besonders bevorzugt in wäßriger Lösung oder Dispersion, umfaßt.

6. Verwendung der pharmazeutischen Zusammensetzung nach einem der Ansprüche 1 bis 5,
**dadurch gekennzeichnet, daß die** pharmazeutische Zusammensetzung in Form einer unviskosierten Lösung vorliegt oder eine Viskosität im Bereich von zwischen 0-7000 mPa·s, vorzugsweise zwischen 2-8 mPa·s aufweist oder in Form eines Gels vorliegt, vorzugsweise mit einer Viskosität im Bereich von zwischen 1000-7000 mPa·s.

7. Verwendung der pharmazeutischen Zusammensetzung nach einem der Ansprüche 1 bis 6,
**dadurch gekennzeichnet, daß** diese in Form eines tropfbaren Gels, einer Salbe oder einer Lösung vorliegt, und ggf. weitere übliche Zusätze, wie Isotonierungsmittel und Substanzen zur Einstellung des pH-Wertes, wie Phosphatpuffer, Boratpuffer, Alkalihydroxid und/oder Säure aufweist.

## Claims

1. Use of a pharmaceutical composition for the production of a medication for the treatment of disorders of the eye caused by bacteria, viruses, fungi, yeasts and/or protozoa, **characterised in that** the pharmaceutical composition comprises as sole pharmaceutically active substance a compound of the general formula or a mixture thereof: 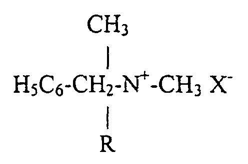 in which R is an alkyl residue of C₈-C₁₈, preferably C₁₂, and X is an anion, preferably a halide, wherein the pharmaceutical composition possibly has a buffer, preferably a phosphate or borate buffer, and also possibly further additives such as water, preservative, EDTA and/or isotonic agent.

2. Use of the pharmaceutical composition according to Claim 1, **characterised in that** the anion is preferably a chloride and/or bromide.

3. Use of the pharmaceutical composition according to Claim 1 or 2, **characterised in that** the alkyl benzyl dimethyl ammonium salt is present in a concentration of ≤ 1% by wt., calculated on the basis of the total pharmaceutical composition, preferably in the range of between 0.00005 % by wt. - 0.2 % by wt., and particularly preferred in the range of between 0.001 % by wt. - 0.05 % by wt.

4. Use of the pharmaceutical composition according to one of Claims 1 to 3, **characterised in that** the pharmaceutical composition comprises substances with osmotic properties such as sodium chloride, sodium nitrate, potassium nitrate, glucose, glycerol and/or sorbitol.

5. Use of the pharmaceutical composition according to one of Claims 1 to 4, **characterised in that** the pharmaceutical composition is rendered non-viscous, preferably comprises at least one viscosity-increasing substance such as synthetic or natural polymer, preferably a cellulose derivative, hydroxy propyl methylcellulose, carbomer, xanthan, dextran, carboxy vinyl polymer, in particular carboxy polymethylene and/or an ethylene-maleic anhydride copolymer and/or hydroxy ethyl cellulose or derivatives as well as mixtures thereof, particularly preferred in aqueous solution or dispersion.

6. Use of the pharmaceutical composition according to one of Claims 1 to 5, **characterised in that** the pharmaceutical composition is present in the form of a solution rendered non-viscous, or has a viscosity in the range of between 0-7000 mPa·s, preferably between 2-8 mPa·s, or is present in the form of a gel, preferably with a viscosity in the range of between 1000-7000 mPa·s.

7. Use of the pharmaceutical composition according to one of Claims 1 to 6, **characterised in that** this is present in the form of a drip gel, an ointment or a solution, and possibly has further usual additives such as isotonic agent and substances to adjust the pH value such as phosphate buffer, borate buffer, alkali hydroxide and/or acid.

## Revendications

1. Utilisation d'une composition pharmaceutique pour la préparation d'un médicament destiné à traiter des affections oculaires causées par des bactéries, des virus, des champignons, des levures et/ou des protozoaires,
**caractérisée en ce que** la composition pharmaceutique comprend comme seule substance pharmaceutiquement active un composé de formule générale ou un mélange de : 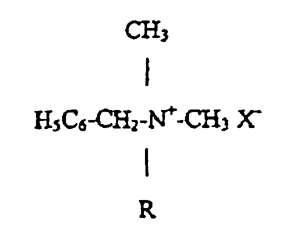 dans laquelle R représente un reste alkyle en C₈-C₁₈, de préférence, en C₁₂ et X représente un anion, de préférence, un halogénure, la composition pharmaceutique présentant, le cas échéant, un tampon, de préférence, un tampon phosphate ou borate ainsi que, le cas échéant, d'autres additifs tels que l'eau, un conservateur, l'EDTA et/ou une solution isotonique.

2. Utilisation de la composition pharmaceutique selon la revendication 1,
**caractérisée en ce que** l'anion est, de préférence, un chlorure et/ou un bromure.

3. Utilisation de la composition-pharmaceutique selon la revendication 1 ou 2,
**caractérisée en ce que** le sel d'alkylbenzyldiméthylammonium est présent dans une concentration de ≤ 1 % en poids rapportée à la totalité de la composition pharmaceutique, de préférence, dans une plage de 0,00005 % en poids à 0,2 % en poids et, de manière particulièrement préférée, dans une plage de 0,001 % en poids à 0,05 % en poids.

4. Utilisation de la composition pharmaceutique selon l'une quelconque des revendications 1 à 3,
**caractérisée en ce que** la composition pharmaceutique comprend des substances présentant des propriétés osmotiques telles que le chlorure de sodium, le nitrate de sodium, le nitrate de potassium, le glucose, le glycérol et/ou le sorbitol.

5. Utilisation de la composition pharmaceutique selon l'une quelconque des revendications 1 à 4,
**caractérisée en ce que** la composition pharmaceutique n'est pas visqueuse, de préférence, elle comprend au moins une substance augmentant la viscosité telle qu'un polymère naturel ou synthétique, de préférence, un dérivé de cellulose, de l'hydroxypropylméthylcellulose, du carbomère, de la gomme xanthane, du dextran, un polymère carboxyvinylique, notamment du carboxypolyméthylène et/ou un copolymère éthylène - anhydride maléique et/ou de l'hydroxyéthylcellulose ou des dérivés, ainsi que des mélanges de ceux-ci, de manière particulièrement préférée, dans une solution ou une dispersion aqueuse.

6. Utilisation de la composition pharmaceutique selon l'une quelconque des revendications 1 à 5,
**caractérisée en ce que** la composition pharmaceutique se présente sous la forme d'une solution non visqueuse ou qu'elle présente une viscosité s'inscrivant dans une plage entre 0 à 7 000 mPa•s, de préférence, entre 2 à 8 mPa•s et qu'elle se présente sous la forme d'un gel, de préférence, présentant une viscosité s'inscrivant dans une plage entre 1 000 à 7 000 mPa•s.

7. Utilisation d'une composition pharmaceutique selon l'une quelconque des revendications 1 à 6,
**caractérisée en ce que** celle-ci se présente sous la forme d'un gel fluide, d'une pommade ou d'une solution et, le cas échéant, qu'elle présente d'autres additifs traditionnels tels qu'une solution isotonique et des substances pour ajuster la valeur du pH telles qu'un tampon phosphate, un tampon borate, un hydroxyde alcalin et/ou des acides.
